Europäisches Patentamt

European Patent Office

Office européen des brevets

(11) Publication number: **0 137 601**
**B1**

## (12) EUROPEAN PATENT SPECIFICATION

(45) Date of publication of patent specification: **25.10.89**

(51) Int. Cl.⁴: **C 12 P 7/62, C 12 N 11/14**

(21) Application number: **84304933.9**

(22) Date of filing: **19.07.84**

(54) Enzymatic synthesis of gallic acid esters.

(30) Priority: **13.10.83 US 541538**

(43) Date of publication of application:
**17.04.85 Bulletin 85/16**

(45) Publication of the grant of the patent:
**25.10.89 Bulletin 89/43**

(84) Designated Contracting States:
**BE DE FR GB NL**

(56) References cited:
FR-A-2 020 527
FR-A-2 078 426

CHEMICAL ABSTRACTS, vol. 65, no. 11, 21st
November 1966, no. 17297c, Columbus, Ohio,
US; W. MADHAVAKRISHNA et al.: "The
essential groups and the nature of crystalline
tannase from divi-divi (Caesalpinia coriaria)
pods", & SEMINAR VEGETABLE TANNINS,
PAPERS, MADRAS 1962, 83-98

(73) Proprietor: **Corning Glass Works**
**Houghton Park**
**Corning New York 14831 (US)**

(72) Inventor: **Weetall, Howard Hayyim**
**4 Deer Run Road**
**Big Flats New York 14814 (US)**

(74) Representative: **Smith, Sydney et al**
**Elkington and Fife Beacon House 113 Kingsway**
**London WC2B 6PP (GB)**

(56) References cited:
CHEMICAL ABSTRACTS, vol. 50, no. 7, 10th
April 1956, no. 5078e, Columbus, Ohio, US; G.
TOTH et al.: "Enzymic synthesis of gallic acid
derivatives", & ACTA CHIM. ACAD. SCI. HUNG.
2, 209-12, 1952

CHEMICAL ABSTRACTS, vol. 81, no. 21, 25th
November 1974, page 124, no. 132218j,
Columbus, Ohio, US; H.H. WEETALL et al.:
"Immobilized tannase", & BIOTECHNOL.
BIOENG. 1974, 16(8), 1095-102

Courier Press, Leamington Spa, England.

# EP 0 137 601 B1

(56) References cited:
CHEMICAL ABSTRACTS, vol. 97, no. 19, 8th
November 1982, no. 158514h, Columbus, Ohio,
US; L.C. KATWA et al.: "Spectrophotometric
assay of immobilized tannase", & J. BIOSCI.
1981, 3(2), 135-42

CHEMICAL ABSTRACTS, vol. 86, no. 17, 25th
April 1977, no. 116829g, Columbus, Ohio, US;
& JP - A - 76 125 790 (KYOWA HAKKO KOGYO
CO., LTD.) 02-11-1976

**Description**

The present invention relates to an improved method of synthesizing gallic acid esters.

The ability of soluble tannase to make gallic acid esters was disclosed by Geza Toth and Deszo Hensler, "The Enzymatic Synthesis of Gallic Acid Derivatives", 10 Acta Chimica II, (1952), pp. 209—212. In this article, Toth et al. describe how soluble tannase can synthesize methyl gallate, ethyl gallate, digallic acid, and glucogallin. In each case, gallic acid and the corresponding ester component, e.g. methanol for methyl gallate, were incubated in the presence of buffered, soluble tannase. After three days, percent synthesis ranged from 0.33% for glucogallin to 33% for methyl gallate.

The present process provides an improved means for synthesizing gallic acid esters. The key difference over the Toth method is the use of an immobilized rather than a soluble tannase. The results of this change are striking. The soluble enzyme worked best with methanol as the ester component, synthesis was 40.1%, but was ineffective with propanol, 3.0% synthesis. However, with the present process, this order is reversed. Methanol and gallic acid produce only a trace of methyl gallate in the present process, while propanol gives in excess of 60% propyl gallate. Thus, the present immobilized tannase method is useful for producing propyl gallate, an antioxidant used heavily in the food processing industry.

Tannase is immobilized by attachment to an insoluble carrier. Both the carriers and the immobilization techniques for tannase are conventional and well known to those of skill in the art. For example, carriers can be made from either siliceous materials, non-siliceous ceramics, or organic polymers including synthetic and natural compounds. Tannase can be attached to these carriers by either adsorption, covalent linkage or entrapment.

The present invention therefore provides an improved method for making gallic acid esters with tannase characterized by:

(a) immobilizing tannase by attachment to an insoluble carrier; and

(b) mixing gallic acid and an ester component of at least three carbon atoms selected from alkyl alcohols and alkyl diols in the presence of the immobilized tannase, whereby the ester component and the gallic acid combine to form a gallic acid ester.

The reactants in the method of the invention are gallic acid and, as ester components of at least three carbon atoms, alkyl alcohols (preferably $C_3$—$C_{12}$) and diols (preferably $C_3$—$C_5$). Gallic acid esters formed by incubating these reactants in the presence of immobilized tannase include propyl, and amyl gallate.

Incubation parameters are fairly broad. Either reactant can be in excess, but usually it is the alcohol reactant. Reaction temperature can vary from 4 to 45°C. Incubation time till equilibration may vary also depending upon the ester component, temperature, and the degree to which one wants to complete the synthesis. Typical times range from 12 to 168 hours.

FIGURE 1 is a graph of the rate of propyl gallate synthesis by the present composite.

FIGURE 2 is a graph of the rate of propyl gallate synthesis by the present composite at different pHs.

Modes of Carrying Out the Invention

Immobilized Tannase

A preferred immobilized tannase composite is based on tannase from *Aspergillus niger* and a porous silica carrier. First the porous silica is derivatized with a α-aminopropyltriethoxy silane. A suitable method is described in U.S. 3,519,538. Then tannase S, a product of *Aspergillus niger*, was covalently coupled to the derivatized porous silica by treating the mixture with glutaraldehyde, as set forth in U.S. 3,669,841. The resulting composite was washed with distilled water, then with a 0.1M $Na_2HPO_4$ (pH 6.0) buffer.

Synthesis of Alkyl Alcohol and Diol Esters

Various alkyl alcohols and diols were reacted with gallic acid. To 10 ml of the alcohol or diol was added 170 mg of gallic acid, thereby forming a 0.1M gallic acid solution. One gram of the above tannase composite was added to 2.0 ml of this solution. The mixture were incubated for 18 hours at room temperature.

Gallic acid ester synthesis was assayed by conventional thin layer chromatography. The incubated mixtures were compared against controls which were incubated without the tannase composite. The coating was Silica Gel No. 13179 (Eastman Kodak, Inc.). The solvent system was 8:2 (vol/vol) chloroform:methanol with 2 drops of glacial acetic acid added per 100 ml of solvent. Visualization was by iodine vapor.

The results of the assay were:

TABLE 1

| Alkyl Alcohol/Dio | $(C_{No.})$ | % Synthesis (Approx.) |
|---|---|---|
| Methanol + | (1) | <10% |
| Ethanol + | (2) | 20% |
| Propanol | (3) | 60—70% |
| Iso-propanol | (4) | 60—70% |
| Butanol | (4) | 70—80% |
| n-amylalcohol | (5) | 85—95% |
| 3 methyl 1-butanol | (5) | 85—95% |
| Octanol | (8) | 20—30% |
| Decanol | (10) | 10—20% |
| Lauryl | (12) | <10% |
| 1,3-propanediol | (3) | 50—60% |
| 1,2-butanediol | (4) | 70—80% |
| 1,3-butanediol | (4) | 70—80% |
| 1,5-pentanediol | (5) | 70—80%* |
| 2,4-pentanediol | (5) | 50—60% |
| 1,4-pentanediol | (5) | 50—60% |

+ These are for purposes of comparison
* In the case of the diols, two monoesters and a diester were formed. These have not been distinguished for percent purposes.

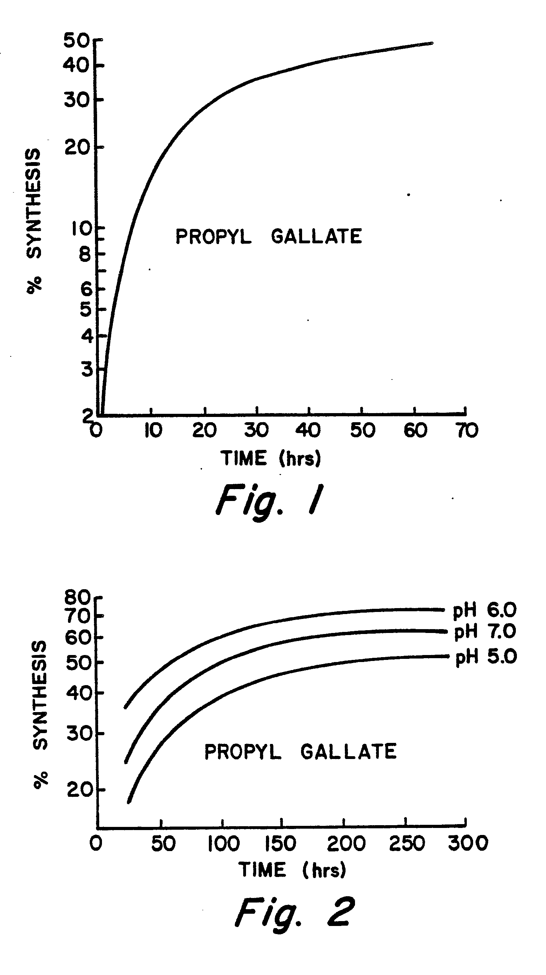

FIGURE 1 illustrated how fast synthesis proceeds with the immobilized tannase. A 0.1M, pH 6.0 gallic acid solution was prepared. One gram of the above tannase composite and 10 ml of propanol were added. As the mixture incubated at room temperature, aliquots were removed and assayed for synthesis.

The effect of pH on ester synthesis was shown in FIGURE 2. The same procedure was used as in the kinetics experiment, except that differently buffered, gallic acid solutions were used. The optimum pH tested was pH 6.0.

Effect of Composite to Reactants Ratio on Synthesis

Propylgallate synthesis was selected to determine the effect of varying the amount of composite while holding the reactant amounts steady. The above Tannase composite and assay method were used herein. Increasing concentrations of immobilized composite (200—800 mg) were added to 2.0 ml of 0.1M gallic acid dissolved in n-propanol. Controls were 0.1M gallic acid in propanol without the composite, and 0.1M propylgallate in propanol.

Incubation occurred at room temperature, and the mixtures were assayed at 18 and 42 hours. The results were that the greater the amount of composite present, the greater the amount of propylgallate synthesized. After 42 hours, propylgallate synthesis was substantially over in the 800 mg sample, but propylgallate was still increasing in concentration in the 200 and 400 mg samples.

Soluble versus Immobilized Tannase

A comparison was conducted between the soluble tannase method of the prior art and the present method.

Soluble enzyme synthesis was run with methanol, ethanol, and propanol. The reactant mixture were as follows:

a) 0.5 g gallic acid
5.0 ml methanol
2.8 mg of Tannase S enzyme in 2.8 ml $H_2O$
7.0 ml of citric acid (pH 5.0) buffer
7.72 ml of $H_2O$;

b) 0.5 g gallic acid
2.0 ml ethanol
2.8 mg of Tannase S enzyme in 2.8 ml $H_2O$
5.0 ml of citric acid (pH 5.0) buffer
12.72 ml $H_2O$; and

c) 0.5 g gallic acid
1.0 ml propanol
2.8 mg of Tannase S enzyme in 2.8 ml of $H_2O$
5.0 ml of citric acid (pH 5.0) buffer
13.72 ml of $H_2O$

Reaction mixture a) essentially duplicated Experiment No. 2 of Toth. In order to match the molarity of ethanol and propanol with that of methanol in Experiment No. 2, the following reaction mixtures were also made:

d) 0.5 g gallic acid
7.24 ml ethanol
2.8 mg of Tannase S enzyme in 2.8 ml of $H_2O$
5.0 ml of citric acid (pH 5.0) buffer
7.48 ml of $H_2O$; and

e) 0.5 g gallic acid
9.46 ml of propanol
2.8 mg of Tannase S enzyme in 2.8 ml of $H_2O$
5.0 ml of citric acid (pH 5.0) buffer
5.26 ml of $H_2O$.

The present process was also tested for degree of synthesis. Here the same amount of enzyme activity present in the Toth mixtures (2.8 mg) is present with the immobilized tannase composite. The reaction mixtures were as follows:

f) 0.1M gallic acid in 2.0 ml amyl alcohol 1.0 g of composite (2.8 mg activity/g);
g) 0.1M gallic acid in 2.0 ml propanol 1.0 g of composite in f);
h) 0.1M gallic acid in 2.0 ml ethanol 1.0 g of composite in f); and
i) 0.1M gallic acid in 2.0 ml ethanol 1.0 g of composite in f).

All of the above mixtures a) — i) were shaken at room temperature for 168 hours. The results were:

TABLE 2

| Mixture | Alcohol | Percent Synthesis |
|---------|---------|-------------------|
| a)* | Methanol | 40.1 |
| i) | Methanol | Trace |
| b)* | Ethanol | 9.4 |
| d)* | Ethanol | 17.9 |
| h) | Ethanol | 3.5 |
| c)* | Propanol | 4.8 |
| e)* | Propanol | 3.0 |
| g) | Propanol | 41.4 |
| f) | Amyl | 78.0 |

*Toth process

# EP 0 137 601 B1

One can clearly see that in the present process the larger the alkyl alcohol, the greater the degree of ester formed. The soluble enzyme method is the reverse. Thus, the present method is the preferred method for synthesizing larger gallic acid esters such as propylgallate.

Having described the invention with particular reference to preferred form, it will be obvious to those skilled in the art to which the invention pertain, that, after understanding the invention, various changes and modifications may be made without departing from the spirit and scope of the invention as defined by the appended claims.

## Claims

1. An improved method for making gallic acid esters with tannase is characterized by:

(a) immobilizing tannase by attachment to an insoluble carrier; and

(b) mixing gallic acid and an ester component of at least three carbon atoms selected from alkyl alcohols and alkyl diols in the presence of the immobilized tannase, whereby the ester component and the gallic acid combine to form a gallic acid ester.

2. A method as claimed in claim 1 characterized in that the immobilized tannase comprises a siliceous carrier derivatized with a silane coupling agent to which a soluble tannase is covalently bonded by means of crosslinking with a dialdehyde.

3. A method as claimed in claim 1 or claim 2 characterized in that stoichiometrically, more ester component is present than gallic acid.

4. A method as claimed in any of claims 1 to 3 characterized in that the ester component, gallic acid, and immobilized tannase are mixed at ambient temperature.

5. A method as claimed in any of claims 1 to 4 characterized in that the ester component is a $C_3$—$C_{12}$ alkyl alcohol.

6. A method as claimed in any of claims 1 to 5 characterized in that the ester component is a $C_3$—$C_5$ alkyl diol.

## Patentansprüche

1. Verbessertes Verfahren zur Herstellung von Gallussäureestern mit Tannase, gekennzeichnet durch

(a) Immobilisieren von Tannase durch Anbringung an einen unlöslichen Träger; und

(b) Vermischen von Gallussäure und einem Esterbestandteil mit wenigstens drei Kohlenstoffatomen, ausgewählt aus Alkylalkoholen und Alkyldiolen, in Gegenwart der immobilisierten Tannase, wobei der Esterbestandteil und die Gallussäure sich verbinden, um einen Gellussäureester zu bilden.

2. Verfahren nach Anspruch 1, dadurch gekennzeichnet, daß die immobilisierte Tannase einen kieselsäurehaltigen Träger aufweist, der mit einem Silan-Kopplungsmittel derivatisiert wurde, an welches eine lösliche Tannase durch Vernetzung mit einem Dialdehyd kovalent gebunden wurde.

3. Verfahren nach Anspruch 1 oder 2, dadurch gekennzeichnet, daß vom Esterbestandteil stöchiometrisch mehr vorhanden ist als von der Gallussäure.

4. Verfahren nach einem der Ansprüche 1—3, dadurch gekennzeichnet, daß der Esterbestandteil, die Gallussäure und die immobilisierte Tannase bei Raumtemperatur vermischt werden.

5. Verfahren nach einem der Ansprüche 1—4, dadurch gekennzeichnet, daß der Esterbestandteil ein $C_3$—$C_{12}$ -Alkylalkohol ist.

6. Verfahren nach einem der Ansprüche 1—5, dadurch gekennzeichnet, daß der Esterbestandteil ein $C_3$—$C_5$ Alkyldiol ist.

## Revendications

1. Un procédé perfectionné pour produire des esters d'acide gallique au moyen de tannase est caractérisé en ce que:

(a) on immobilise de la tannase par fixation à un support insoluble; et

(b) on mélange l'acide gallique et un composant d'ester ayant au moins trois atomes de carbone choisi parmi les alcools alkyliques et les diols alkyliques en présence de la tannase immobilisée, de sorte que le composant d'ester et l'acide gallique se combinent pour former un ester d'acide gallique.

2. Un procédé tel que revendiqué dans la revendication 1, caractérisé en ce que la tannase immobilisée comprend un support silicieux transformé en dérivé par un silane servant d'agent de couplage auquel une tannase soluble est liée par covalence au moyen de liaisons transversales formées par un dialdéhyde.

3. Un procédé tel que revendiqué dans la revendication 1 ou la revendication 2, caractérisé en ce qu'il y a, stoechiométriquement, davantage de composant d'ester que d'acide gallique.

4. Un procédé tel que revendiqué dans l'une quelconque des revendications 1 à 3, caractérisé en ce que le composant d'ester, l'acide gallique et la tannase immobilisée sont mélangés à la température ambiante.

5. Un procédé tel que revendiqué dans l'une quelconque des revendications 1 à 4, caractérisé en ce que le composant d'ester est un alcool alkylique en $C_3$—$C_{12}$.

6. Un procédé tel que revendiqué dans l'une quelconque des revendications 1 à 5, caractérisé en ce que le composant d'ester est un diol alkylique en $C_3$—$C_5$.

EP 0 137 601 B1

% SYNTHESIS

PROPYL GALLATE

TIME (hrs)

*Fig. 1*

% SYNTHESIS

pH 6.0
pH 7.0
pH 5.0

PROPYL GALLATE

TIME (hrs)

*Fig. 2*

1